# EUROPÄISCHE PATENTSCHRIFT

(11) **EP 2 626 049 B1**
(45) Veröffentlichungstag und Bekanntmachung des Hinweises auf die Patenterteilung: **25.07.2018**
(21) Anmeldenummer: 12000901.4
(22) Anmeldetag: 11.02.2012
(51) Int. Cl.: A61M 3/02, A61M 13/00, A61F 13/00, A61F 13/02, A61M 35/00, A61M 1/00

(54) **WUNDTHERAPIEVORRICHTUNG**
WOUND TREATMENT DEVICE
DISPOSITIF POUR LA THÉRAPIE DES PLAIES

(43) Veröffentlichungstag der Anmeldung: 14.08.2013
(73) Patentinhaber: Paul Hartmann AG, 89522 Heidenheim (DE)
(72) Erfinder: Croizat, Pierre, 89542 Herbrechtingen (DE); Eckstein, Axel, 89522 Heidenheim (DE)

(56) Entgegenhaltungen:
- US-A1- 2006 025 727
- US-A1- 2007 032 763
- US-A1- 2008 234 641
- US-A1- 2009 030 383
- US-A1- 2009 054 855
- US-A1- 2010 087 791
- US-A1- 2011 015 587
- US-B1- 6 398 767

## Beschreibung

Die vorliegende Erfindung betrifft eine Vorrichtung zur Verwendung bei der Unterdrucktherapie von Wunden, insbesondere eine Vorrichtung für die Behandlung von Wunden mittels Unterdruck und Fluidspülung. Die Erfindung betrifft weiterhin ein Verfahren zur Dosierung eines geeigneten Volumens an Instillfluid für die Instilltherapie sowie ein Verfahren zur Bestimmung eines geeigneten Volumens an Instillfluid für die Instilltherapie von Wunden.

Unter dem Begriff Unterdrucktherapie wird dabei ein Verfahren zur Behandlung von Wunden verstanden, bei dem Unterdruck im Wundraum appliziert wird und Flüssigkeit aus einer Wunde abgesaugt wird. Unter Wundraum wird der zwischen einer fluiddichten Abdeckfolie und dem Wundgrund (Körpergewebe im Wundbereich) gebildete Zwischenraum verstanden.
Der Ausdruck "Unterdruck" bezeichnet dabei einen im Wundraum gegenüber dem Umgebungsluftdruck (atmosphärischer Luftdruck) erniedrigten Luftdruck. "Unterdruck" wird häufig auch als "negativer Druck" bezeichnet. Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck im Wundraum und dem Umgebungsluftdruck, als positiver Zahlenwert in mm Hg angegeben. Eine Erhöhung des Unterdrucks bedeutet folglich eine Vergrößerung der Druckdifferenz zwischen dem Luftdruck im Wundraumes und dem Umgebungsluftdruck. Eine Verringerung des Unterdrucks bedeutet folglich eine Verminderung der Druckdifferenz zwischen dem Luftdruck im Wundraumes und dem Umgebungsluftdruck.

Vorrichtungen zur Unterdrucktherapie von Wunden und Wundauflagen als Bestandteil derartiger Vorrichtungen sind im Stand der Technik bekannt.
So beschreibt beispielsweise die WO1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine als Dichtungseinrichtung bezeichnete luftdichte Abdeckung der Wunde, welche mit der Vakuumeinrichtung in einer funktionellen Verbindung steht, sowie eine als Schutzeinrichtung bezeichnete Wundauflage zur Positionierung an der Wunde innerhalb der Dichtungseinrichtung. Bei der Schutzeinrichtung handelt es sich um einen porösen Polymerschaumstoff, beispielsweise um Polyester-Schaum. Ausweislich der Beschreibung von WO1993/009727 kann durch die Anwendung der Unterdrucktherapie die Wundheilung unterschiedlich Typen von Wunden wie beispielsweise Brandwunden oder Druckwunden beschleunigt werden.

Die Unterdrucktherapie von Wunden unter gleichzeitiger oder abwechselnder Spülung der Wunde mit einem Fluid (Instillfluid) wird auch als Instilltherapie bezeichnet. Im Rahmen der vorliegenden Patentanmeldung wird der Begriff Instillfluid als Oberbegriff für Instillflüssigkeiten und Instillgase verwendet.

Im Stand der Technik sind Unterdrucktherapiesysteme zur Behandlung von Wunden bekannt, bei denen eine Spülung der Wunde mit einem Instillfluid möglich ist. Eine derartige Vorrichtung wird auch als Instilltherapievorrichtung bezeichnet. Beispielhaft wird hierzu auf die WO2001/037922 verwiesen, die eine Instilltherapievorrichtung für die Behandlung von Wunden mittels Unterdruck und Fluidspülung beschreibt.

Als Instillflüssigkeiten werden häufig Wasser, Kochsalzlösungen, insbesondere isotone Kochsalzlösungen, oder medizinische Lösungen wie Ringerlösung verwendet. Die Instillflüssigkeiten können Wirkstoffe wie z.B. antiseptische Stoffe, Antibiotika, Wachstums- und Blutgerinnungsfaktoren oder Schmerzmittel enthalten.

Als Instillgase werden häufig Luft, mit Sauerstoff angereicherte Luft oder Stickstoff eingesetzt.

Ein wesentlicher Vorteil der Instilltherapie gegenüber einer herkömmlichen Unterdrucktherapie ist es, dass die Wunde ohne Verbandswechsel und ohne längere Unterbrechung der Unterdrucktherapie mit einem Instillfluid gespült werden kann. Das Instillfluid kann gegebenenfalls für eine bestimmte Zeit in der Wunde verbleiben und wird dann mittels Unterdruck abgesaugt. Typische Zeiträume sind einige Minuten bis Stunden. Während des Verbleibens des Instillfluids in der Wunde kann das Instillfluid z.B. nekrotisches Gewebe und Zelltrümmer lösen. Auf diese Weise kann die Instilltherapie zu einem Debridement der Wunde beitragen. Weiterhin kann das Instillfluid ein die Wundheilung begünstigendes Wundmillieu fördern, beispielsweise durch Beeinflussung des pH-Werts der Wunde oder durch eine Verbesserung der Sauerstoffversorgung. Insbesondere kann ein Wundmillieu im sauren pH-Bereich förderlich für die Wundheilung sein. Optional kann das Instillfluid auch Wirkstoffe umfassen, welche die Wundheilung unterstützen.

Bei der Instilltherapie von Wunden stellt sich stets das Problem der Dosierung des Instillfluid. Bei einem zu geringem Volumen an zugeführtem Instillfluid kann die im Zusammenhang mit der Fluidspülung erwünschte Wirkung ausbleiben. Weiterhin kann ein zu geringes Volumen an Instillfluid dazu führen, dass in dem Instillfluid enthaltene Wirkstoffe wie z.B. Antibiotika oder Wachstumsfaktoren in zu geringer Menge in der Wunde vorliegen. Der gewünschte wundheilungsfördernde Effekt kann dann nicht oder nur unzureichend erzielt werden. Ein zu großes Volumen an zugeführtem Instillfluid kann zu einem Überdruck innerhalb des Wundverbandes führen. Ein solcherart im Wundraum erzeugter Überdruck kann die Wunde schädigen und zur Mazeration der intakten, die Wunde umgebenden Haut führen. Zudem kann ein Überdruck zum Überdehnen oder Aufblähen des Verbandes führen, was den Sitz des Verbandes auf der Wunde beeinträchtigt. Ein Überdruck im Wundraum kann weiterhin auch zu einer Undichtigkeit des Verbandes führen, so dass der Unterdruckverband vorzeitig gewechselt werden muss. Ein zusätzlicher Wechsel der Wundauflage kann die heilende Wunde traumatisieren und mit Schmerzen für den Patienten verbunden sein. Ein Verbandwechsel, welcher bei schweren Wunden häufig im Operationssaal durchgeführt werden muss, stellt zudem einen erheblichen Zeitaufwand für das medizinische Personal dar und ist mit zusätzlichen Kosten verbunden.

Die Menge an Instillfluid kann beispielsweise durch Einleiten des Instillfluids und visuelle Kontrolle der Aufwölbung der Wundauflage kontrolliert werden. Solchenfalls erkennt der Benutzer einer Instilltherapievorrichtung an einer beginnenden Aufwölbung der Wundauflage, dass genug Instillfluid eingeleitet wurde. Die Zufuhr weiteren Instillfluids muss dann unterbrochen werden. Nachteilig an diesem Verfahren zur Dosierung der benötigten Menge an Instillfluid ist es, dass die Einschätzung des für die Unterbrechung einer weiteren Instillfluid-Zufuhr geeigneten Zeitpunktes subjektiv durch den Benutzer erfolgt und daher von Fall zu Fall unterschiedlich ausfallen kann.

Die WO2010/033272A1 beschreibt eine Vorrichtung, welche für das Spülen einer Wunde mit einer Spülflüssigkeit geeignet ist. Gemäß dem in der WO2010/033272A1 beschriebenen Behandlungsverfahren strömt die Spülflüssigkeit durch die Wunde und wird zusammen mit Wundflüssigkeit (Wundexsudat) in einen Behälter geleitet. Die Spülflüssigkeit ist bevorzugt hyperton und bewirkt mittels Osmose ein Einströmen von Körperflüssigkeit in die Wunde. Das Volumen der aus der Wunde abgeleiteten Flüssigkeit kann mit einem Messgerät am Behälter festgestellt werden, um die Therapie zu überwachen. Aus der Differenz zwischen dem Volumen der in die Wunde eingeleiteten Spülflüssigkeit und dem gemessenen Volumen ergibt sich die Menge der abgeleiteten Wundflüssigkeit.

Die WO2009/071924A1 offenbart ein Verfahren zur Bestimmung des Volumens einer Wunde, bei der die Luftmenge gemessen wird, die zur Erzeugung eines bestimmten Unterdrucks aus der Wunde abgesaugt werden muss. Die Bestimmung der Luftmenge gestattet die Berechnung oder Abschätzung des Wundvolumens. Das derart ermittelte Wundvolumen dient zur Dosierung eines geeigneten Volumens an Instillfluid. Ein Nachteil bei dieser Methode ist, dass das Wundvolumen nur indirekt über die aus der Wunde entfernte Luftmenge bestimmt werden kann. Da es sich bei Luft um ein komprimierbares Gas handelt, kann die gemessene Luftmenge in Abhängigkeit vom Unterdruck bei gleichem Wundvolumen schwanken. Weiterhin hängt die Berechnung des Wundvolumens von veränderlichen Parametern wie der Zusammensetzung der Luft, der Luftfeuchtigkeit und der Temperatur der Luft ab.

Der vorliegenden Erfindung liegt daher die Aufgabe zugrunde, eine verbesserte Methode zur Instilltherapie von Wunden bereit zu stellen, welche die genannten Nachteile des Standes der Technik überwindet. Insbesondere soll die Dosierung und die Bestimmung eines geeigneten Volumens an Instillfluid verbessert werden. Die Dosierung und Bestimmung eines geeigneten Volumens an Instillfluid soll zudem vereinfacht und automatisiert werden.

Als Lösung dieser Aufgabe schlägt die Erfindung eine Wundtherapievorrichtung für die Behandlung von Wunden mittels Unterdruck und Fluidspülung nach Anspruch 1 vor. Die erfindungsgemäße Vorrichtung umfasst demnach einen Unterdruckverband mit einer fluiddichten Abdeckung, so dass ein Wundraum zwischen der Abdeckung und dem Wundgrund gebildet werden kann, ein Drucksensormittel, welches Druckmesswerte erzeugen kann, in Fluidkommunikation mit dem Wundraum, einen Drainagebehälter zur Sammlung von Flüssigkeit und eine Drainageleitung, mittels derer Flüssigkeit aus dem Wundraum in den Drainagebehälter geleitet werden kann. Die Vorrichtung umfasst weiterhin eine erste Pumpeneinheit zum Absaugen von Flüssigkeit aus dem Wundraum über die Drainageleitung, wobei die erste Pumpeneinheit fluidleitend mit dem Drainagebehälter verbunden ist. Weiter umfasst die Vorrichtung einen Instillbehälter zur Bereitstellung eines Instillfluids, eine Instillleitung, mittels derer Instillfluid aus dem Instillbehälter in den Wundraum zugeführt werden kann, ein Mittel zum Zuführen eines Instillfluids aus dem Instillbehälter in den Wundraum und ein Steuerungsmittel, wobei das Steuerungsmittel Druckmesswerte vom Drucksensormittel empfangen kann. Erfindungsgemäß ist die Vorrichtung dadurch gekennzeichnet, dass das Steuerungsmittel bei Erreichen eines voreingestellten oberen Unterdruck-Schwellenwertes im Wundraum ein Signal zum Abschalten der ersten Pumpeneinheit ausgeben kann, und dass das Steuerungsmittel bei Erreichen eines voreingestellten unteren Unterdruck-Schwellenwertes im Wundraum ein Signal zum Unterbrechen der Zufuhr des Instillfluids ausgeben kann. Unter einem "oberen Unterdruck-Schwellenwert" wird hierbei ein Schwellenwert in demjenigen Unterdruckbereich verstanden, welcher üblicherweise zu einer Unterdrucktherapie eingesetzt wird, beispielsweise 13332,2 Pa (100 mm Hg) oder 16665,25 Pa (125 mm Hg). Unter einem "unteren Unterdruck-Schwellenwert" wird dagegen ein Schwellenwert verstanden, welcher im Vergleich zum "oberen Unterdruck-Schwellenwert" näher am Umgebungsluftdruck liegt, beispielsweise 0 Pa (0 mm Hg) oder 3333,05 Pa (25 mm Hg).

Ein Unterdruckverband im Sinne der Erfindung ist ein für die Unterdrucktherapie von Wunden geeigneter Verband, welcher einen Wundraum gasdicht abschließen kann und eine Fluidkommunikation mit einer Unterdruckquelle und mit einer Instillfluidquelle ermöglicht. Der Unterdruckverband umfasst eine fluiddichte Abdeckung. Die Drainage- und/oder Instillleitung kann im einfachsten Falle unter dem Rand der fluiddichten Abdeckung oder durch die Abdeckung hindurch in den Wundraum geführt werden. Vorzugsweise ist mindestens ein Mittel zum Anschluss einer Drainage- und/oder Instillleitung vorhanden. Insbesondere handelt es sich bei dem mindestens einen Mittel zum Anschluss einer Drainage- und/oder Instillleitung um ein Unterdruckanschlussstück (Port). Geeignete Unterdruckanschlussstücke sind beispielsweise in den Patentanmeldungen WO2011/076340, WO2011/091952 oder in der unveröffentlichten Patentanmeldung DE102011108726.9 des Anmelders Paul Hartmann AG beschrieben. Ein Unterdruckanschlussstück, welches einen gleichzeitigen Anschluss von Drainageleitung und Instillleitung erlaubt, ist in der unveröffentlichten Patentanmeldung DE102011082341.7 des Anmelders Paul Hartmann AG offenbart.

Bei der fluiddichten Abdeckung handelt es sich üblicherweise um eine selbstklebende Abdeckfolie. Im Zusammenhang mit der Erfindung kann beispielsweise das Fertigprodukt Hydrofilm® (Paul Hartmann AG, Heidenheim) als geeignete Abdeckfolie verwendet werden.
Weiterhin umfasst der Unterdruckverband üblicherweise eine Wundauflage, beispielsweise eine textile Wundauflage oder einen Schaumstoff, insbesondere einen offenzelligen Polymerschaumstoff. Die Wundauflage kann einlagig oder mehrlagig ausgebildet sein. So können beispielsweise die Handelsprodukte VivanoMed® Foam, TenderWet® oder ES-Kompresse der Firma Paul Hartman AG (Heidenheim) einzeln oder in Kombination als Wundauflage eingesetzt werden. Die Wundauflage kann eine zusätzliche Wundkontaktschicht aufweisen, beispielsweise Salbenwundauflage, insbesondere eine antimikrobiell ausgerüstete Salbenwundauflage. Bei der Salbenwundauflage kann es sich vorzugsweise um ein versilbertes Polyamidgewirke handeln. Ein versilbertes Polyamidgewirke ist unter der Bezeichnung Atrauman Ag® vom Hersteller Paul Hartman AG, Heidenheim, kommerziell erhältlich. Weitere für eine zusätzliche Wundkontaktschicht geeignete Handelsprodukte sind die Wundauflagen Branolind®, Grassolind® oder Hydrotüll® der Firma Paul Hartmann AG.

Das Drucksensormittel steht in Fluidkommunikation mit dem Wundraum und kann Druckmesswerte erzeugen. Das Drucksensormittel kann dabei einen oder mehrere Drucksensoren, insbesondere miniaturisierte elektronische Drucksensoren, umfassen. Ist mehr als ein Drucksensor vorhanden, so können die Sensoren gegebenenfalls an unterschiedlichen Positionen der Wundtherapievorrichtung vorhanden sein. Als Drucksensor kann grundsätzlich jeder Sensor verwendet werden, welcher zur Bestimmung des Drucks eines Fluids geeignet ist. Das Drucksensormittel muss so ausgelegt sein, dass eine Erzeugung und eine Weitergabe von Druckmesswerten über eine Kommunikationsverbindung möglich ist.

Eine Kommunikationsverbindung im Sinne der Erfindung kann dabei jede Art von geeigneter elektrischer Signalübertragungsvorrichtung sein, beispielsweise ein Stromkabel, ein USB-Kabel, ein Kabel für serielle Datenübertragung oder ein Kabel für parallele Datenübertragung. Die Kommunikationsverbindung kann auch eine Funkverbindung sein, beispielsweise eine W-LAN Verbindung oder eine Bluetooth-Verbindung. Weiterhin kann die Kommunikationsverbindung auch auf optische Signale basieren, beispielsweise auf Lichtsignalen, welche in einem Glasfaserkabel übertragen werden. Die Kommunikationsverbindung kann gleichfalls Schnittstellen für die Informationsübertragung mit Infrarotem Licht oder eine Machine-to Machine-Verbindung umfassen.

Die Vorrichtung umfasst einen Drainagebehälter zur Sammlung von Flüssigkeit, welche mittels des Unterdrucks aus der Wunde bzw. aus dem Wundraum abgeleitet wird. Bei der im Drainagebehälter gesammelten Flüssigkeit kann es sich sowohl um Wundflüssigkeit als auch um im Wundraum befindliche Instillflüssigkeit handeln. Vorzugsweise ist der Behälter ein flüssigkeits- und gasdichtes Gefäß, welches sich bei den in der Unterdruck- und Instilltherapie vorherrschenden Druckverhältnissen nicht wesentlich verformt, beispielsweise ein Kunststoffkanister. Der Drainagebehälter ist vorzugsweise ein Einmalartikel, welcher nach Gebrauch entsorgt wird.

Die Vorrichtung umfasst eine Drainageleitung, mittels derer Flüssigkeit aus dem Wundraum in den Drainagebehälter geleitet werden kann. Unter einer Leitung, beispielsweise einer Drainageleitung, einer Instillleitung oder einer Spüllleitung, wird im Rahmen der vorliegenden Erfindung jede Art von fluidleitender Verbindung verstanden. Vorzugsweise ist eine Leitung ein flüssigkeits- und gasdichter Schlauch. Bevorzugt ist ein solcher Schlauch aus Kunststoff gefertigt, insbesondere aus Silikon. Flach und biegsam ausgebildete Schläuche haben sich in der Therapie besonders bewährt, da derartige flache Leitungen weniger Schmerzen bereiten, wenn der Patient auf ihnen liegt.

Der Unterdruck im Wundraum wird durch eine erste Pumpeneinheit erzeugt. Eine Pumpeneinheit umfasst eine übliche, für medizinische Anwendungen geeignete Pumpe. Vorzugsweise umfasst die Pumpeneinheit eine Membranpumpe, wie z.B. Membranpumpen der Hersteller KNF (Freiburg, Deutschland) oder Thomas (Puchheim, Deutschland) Insbesondere ist das Pumpenmodel 1420VP-6VDL der Firma Thomas geeignet. Ein wesentlicher Vorteil einer Membranpumpe besteht darin, dass bei dieser Konstruktionsform keine Teile der Pumpe mit dem Fluid in Kontakt kommen. Dadurch wird die Kontamination der Pumpeneinheit mit Mikroorganismen stark vermindert, die Reinigung der Pumpeneinheit wird erleichtert und die Lebensdauer verlängert. Denkbar und vorteilhaft wäre auch der Einsatz einer Peristaltikpumpe. Der im Wundraum vorhandene Unterdruck bewirkt ein Absaugen von Fluid (Wundexsudat und gegebenenfalls Instillfluid) aus der Wunde. Das Fluid gelangt über die Drainageleitung in den Drainagebehälter. Hierzu ist die erste Pumpeneinheit fluidleitend mit dem Drainagebehälter verbunden. Vorzugsweise ist der Drainagebehälter im Fluidweg zwischen der ersten Pumpeneinheit und der Drainageleitung angeordnet. Die erste Pumpeneinheit befindet sich solchenfalls stromabwärts des Drainagebehälters, so dass die Pumpeneinheit den Unterdruck durch den Drainagebehälter hindurch über die Drainageleitung dem Wundverband zur Verfügung stellt. Hierbei sollte möglichst zwischen Drainagebehälter und Pumpeneinheit ein bei vollem Behälter automatisch schließender Schutzfilter oder Schutzventil angebracht sein, um eine Kontamination der Pumpe mit Wundflüssigkeit zuverlässig zu verhindern. Wundseitig ist die Drainageleitung vorzugsweise mittels eines Unterdruckanschlussstücks (Port) am Unterdruckverband befestigt. Der Port ermöglicht neben der Befestigung der Leitung die Herstellung einer Fluidkommunikation zwischen Drainageleitung und Wundraum.

Das Steuerungsmittel kann die erste Pumpeneinheit kontrollieren, d.h. es kann die erste Pumpeneinheit einschalten, abschalten und die Pumpenhubfrequenz steuern. Die Kontrolle des Steuerungsmittels über die erste Pumpeneinheit erfolgt insbesondere mittels Steuerungssignalen. Weiter kann das Steuerungsmittel Druckmesswerte von dem Drucksensormittel empfangen und auf Basis der Druckmesswerte den Unterdruck im Wundraum ermitteln. Dies ermöglicht eine permanente Überwachung des Unterdrucks im Wundraum durch das Steuerungsmittel. Entsprechend kann der Verlauf der Unterdrucktherapie durch das Steuerungsmittel präzise gesteuert werden. Insbesondere kann das Steuerungsmittel bei Erreichen eines voreingestellten oberen Unterdruck-Schwellenwertes im Wundraum ein Signal zum Abschalten der ersten Pumpeneinheit ausgeben, um das Erzeugen eines zu hohen Unterdrucks in der Wunde zu vermeiden. Ein zu hoher Unterdruck kann die Wunde schädigen und die Wundheilung behindern. Vorzugsweise wird der Benutzer der Wundtherapievorrichtung durch geeignete Anzeigemittel über den angelegten Unterdruck und/oder den zeitlichen Verlauf der Unterdrucktherapie informiert werden. Mittels geeigneter Eingabemittel wie beispielsweise Tasten, einer Tastatur, einem Touchscreen oder einem Computerinterface können komplexe Programme zur zeitgesteuerten Unterdrucktherapie eingegeben. Besonders vorteilhaft für die Wundheilung ist es, die Unterdrucktherapie intermittierend auszuführen. Bei einer intermittierenden Unterdrucktherapie wird die Höhe des applizierten Unterdrucks in regelmäßigen Abständen variiert. So wird der Unterdruck beispielsweise nach Verstreichen einer vorher festgelegten Zeitspanne abgesenkt. Nach Verstreichen einer vorher festgelegten weiteren Zeitspanne wird der Unterdruck wieder erhöht.

Die erfindungsgemäße Wundtherapievorrichtung weist einen Instillbehälter zur Bereitstellung eines Instillfluids auf. Bei dem Instillbehälter kann es sich beispielsweise um einen Kanister oder um einen flüssigkeitsundurchlässigen Beutel handeln. Hierbei können grundsätzlich Mehrwegbehälter oder Einwegbehälter eingesetzt werden. Vorzugsweise ist der Instillbehälter ein Einwegbehälter, insbesondere ein herstellerseitig mit einer sterilen Instillfluid gefüllter Einwegbehälter. Der Instillbehälter kann über die Instillleitung fluidleitend mit dem Wundraum verbunden werden. Weiterhin umfasst die erfindungsgemäße Vorrichtung ein Mittel zum Zuführen eines Instillfluids aus dem Instillbehälter in den Wundraum. Vorzugsweise handelt es sich bei dem Mittel um eine zur Förderung von Flüssigkeiten geeignete zweite Pumpeneinheit (Fluid-Förderpumpe). Die Fluid-Förderpumpe umfasst vorzugsweise eine Membranpumpe oder eine Peristaltikpumpe.

Die Wundtherapievorrichtung der vorliegenden Erfindung zeichnet sich weiter dadurch aus, dass das Steuerungsmittel bei Erreichen eines voreingestellten unteren Unterdruck-Schwellenwertes im Wundraum ein Signal zum Unterbrechen der Zufuhr des Instillfluids ausgeben kann. Eine Unterbrechung der Zuführung von Instillfluid verhindert, dass in der Wunde durch weiteres Einleiten von Instillfluid ein Druck erzeugt wird, welcher den Umgebungsdruck wesentlich übersteigt. Ein Druck oberhalb des Umgebungsdruckes könnte die Wunde schädigen und die Wundheilung beeinträchtigen. Die Unterbrechung der Zuführung von Instillfluid gemäß dem erfindungsgemäß vorgeschlagenen Verfahren stellt gleichzeitig sicher, dass bei der Instilltherapie ein geeignetes Volumen an Instillfluid in die Wunde eingebracht wird.

Nach dem Einbringen eines geeigneten Volumens an Instillfluid entspricht der Druck im Wundraum weitgehend dem Umgebungsdruck. Ein weitgehende Annäherung des Unterdrucks im Wundverband an den Umgebungsdruck hat bei der Verwendung einer Wundauflage aus einem offenzelligen Polyurethanschaum (beispielsweise der Schaum VivanoMed Foam® der Firma Paul Hartmann AG) in der Regel dann stattgefunden, wenn das Volumen des in die Wunde eingeleiteten Instillfluids zwei von drei Teilen (2/3) des Volumens des durch eine Abdeckfolie abgeschlossenen Wundraums beträgt.

Die vorliegende Erfindung umfasst weiter ein Verfahren zur Dosierung eines geeigneten Volumens an Instillfluid bei der Instilltherapie von Wunden. Das Verfahren zur Dosierung eines geeigneten Volumens an Instillfluid wird durchgeführt, indem zunächst eine erfindungsgemäße Wundtherapievorrichtung zur Verfügung gestellt wird. Nach dem Anlegen des Unterdruckverbandes an die Wunde sowie des Anschluss von Drainage- und Instillleitung wird durch Anschalten der ersten Pumpeneinheit ein Unterdruck im Wundraum erzeugt. Dabei werden gegebenenfalls im Wundraum vorhandene Flüssigkeiten durch die Drainageleitung in den Drainagebehälter gesaugt. Der Druck im Wundraum wird mittels eines Drucksensormittels überwacht. Beim Erreichen eines oberen Unterdruck-Schwellenwertes, beispielsweise 13332,2 Pa (100 mm Hg) oder 16665,25 Pa (125 mm Hg), wird die erste Pumpeneinheit abgeschaltet. Nach dem Abschalten der ersten Pumpeneinheit wird Instillfluid aus dem Instillbehälter durch die Instillleitung in den Wundraum bis zur Erreichung eines unteren Unterdruck-Schwellenwertes, beispielsweise 1333,22 Pa (10 mm Hg) oder 2666,44 Pa (20 mm Hg), zugeführt. Die weitere Zuführung von Instillfluid in die Wunde wird bei Erreichung eines unteren Unterdruck-Schwellenwertes insbesondere dadurch beendet, indem das Mittel zum Zuführen des Instillfluids inaktiviert wird und/oder indem ein zwischen Instillbehälter und Wundverband angeordnetes Ventil die weitere Zufuhr von Instillfluid unterbricht.

Vorzugsweise umfasst das erfindungsgemäße Verfahren die Bestimmung eines geeigneten Volumens an Instillfluid für die Instilltherapie von Wunden und die Speicherung des ermittelten Volumens. Wird nämlich ein geeignetes Volumen an Instillfluid bei Beginn der Instilltherapie (erster Spülzyklus) appliziert, so ist es vorteilhaft, wenn beispielsweise ein ebensolches Volumen an Instillfluid oder ein vorher festgelegter Anteil des ursprünglichen Volumens bei nachfolgenden Spülzyklen in die Wunde appliziert wird. Hierzu wird vorgeschlagen, dass das im ersten Spülzyklus erfindungsgemäß dosierte Volumen an Instillfluid ermittelt und zur Dosierung weiterer Spülzyklen zumindest vorübergehend gespeichert wird. Die Bestimmung eines geeigneten Volumens an Instillfluid kann beispielsweise erfolgen, indem die Dauer der ersten Instillfluid-Zufuhr, also die Zeitspanne bis zum Erreichen des unteren Schwellenwertes, ermittelt wird. Bei bekannter Förderleistung des Mittels zum Zuführen des Instillfluids kann das bis zur Unterbrechung der Instillfluid-Zufuhr applizierte Volumen an Instillfluid leicht berechnet werden.
Eine erneute Bestimmung eines geeigneten Volumens an Instillfluid ist solchenfalls erst nach einem Verbandswechsel erforderlich.

Die vorliegende Erfindung umfasst gemäß einer besonders bevorzugten Ausführungsform eine Wundtherapievorrichtung, welche zur Durchführung des vorstehend beschriebenen Verfahrens zur Dosierung eines geeigneten Volumens an Instillfluid bei der Instilltherapie von Wunden geeignet ist. Insbesondere umfasst die vorliegende Erfindung eine Wundtherapievorrichtung, welche zur Durchführung des vorstehend beschriebenen Verfahrens zur Bestimmung eines geeigneten Volumens an Instillfluid für die Instilltherapie und die Speicherung des ermittelten Volumens geeignet ist.

Das Drucksensormittel kann im Wundraum angeordnet sein, also in dem zwischen Abdeckfolie und Wundgrund gebildeten Zwischenraum. Das Drucksensormittel, beispielsweise ein oder mehrere Drucksensoren, kann dabei in eine Wundauflage, beispielsweise in eine Wundauflage aus offenzelligen Polyurethanschaum, integriert sein.
Überraschenderweise hat es sich gezeigt, dass die Bestimmung des im Wundraum vorhandenen Unterdrucks auch dann sehr genau erfolgen kann, wenn das Drucksensormittel an einem beliebigen Ort der Vorrichtung vorgesehen ist, solange das Drucksensormittel in Fluidkommunikation mit dem Wundraum steht.
Die Bestimmung des Unterdrucks im Wundraum erfolgt auf Basis der vom Drucksensormittel an das Steuerungsmittel weitergegebenen Druckmesswerte. Um die Genauigkeit der Bestimmung des Unterdrucks im Wundraum zu verbessern, kann gegebenenfalls eine zusätzliche Verarbeitung der Druckmesswerte, beispielsweise unter Verwendung eines Korrekturalgorithmus, erfolgen.

Die Fluidkommunikation des Drucksensormittels mit dem Wundraum kann beispielsweise über die Drainageleitung realisiert werden. So wäre es beispielsweise möglich, dass ein oder mehrere Drucksensoren in der Drainageleitung oder in einem von der Drainageleitung abzweigenden Leitungsstück angeordnet sind. Gleichfalls ist es möglich, dass mindestens ein Drucksensor im Drainagebehälter oder in einem von vom Drainagebehälter abzweigenden Leitungsstück angeordnet ist.
Falls die erste Pumpeneinheit stromabwärts des Drainagebehälters angeordnet ist, so ist es insbesondere möglich, dass das Drucksensormittel in einem zwischen Drainagebehälter und erster Pumpe angeordneten Leitungsabschnitt oder in einem von dieser Leitung abzweigenden Leitungsstück angeordnet ist.

Gemäß einer weiteren bevorzugten Ausführungsform weist die Wundtherapievorrichtung eine separate Druckmessleitung auf, wobei die Druckmessleitung in Fluidkommunikation mit dem Wundraum und mit dem Drucksensormittel steht.

Gemäß einer weiteren besonders bevorzugten Ausführungsform weist die Wundtherapievorrichtung eine Belüftungsleitung oder eine Spülleitung auf, wobei die Belüftungsleitung oder die Spülleitung in Fluidkommunikation mit dem Wundraum und mit dem Drucksensormittel steht.

Unter einer Belüftungsleitung wird hierbei eine zusätzliche, zum Unterdruckverband und in den Wundraum führende Leitung verstanden, mittels derer zur Druckangleichung oder zur Durchspülung des Fluidsystems Umgebungsluft in den Wundraum eingeführt werden kann. Die zugeführte Umgebungsluft sollte dabei durch einen Bakterienfilter geleitet werden, um eine Kontamination des Wundraums mit Keimen auszuschließen. Der Bakterienfilter kann beispielsweise an dem von der Wunde abgewandten Ende der Belüftungs- oder Spülleitung vorhanden sein. Die Belüftungs- oder Spülleitung steht mit ihrem wundseitigen Ende in Fluidkommunikation mit dem Wundraum. Das von der Wunde abgewandte Ende der Belüftungsleitung oder Spülleitung steht über ein regelbares Belüftungsventil oder Spülventil mit der Umgebung in Fluidkommunikation, so dass eine Belüftung oder Durchspülung des Wundraums und der Drainageleitung ermöglicht wird, wenn das Ventil einen Öffnungsimpuls erhält. Bei dem Belüftungsventil oder Spülventil handelt es sich normalerweise um ein elektrisch zu betätigendes Ventil, vorzugsweise um ein magnetisches Ventil, welches mit dem Steuerungsmittel kommuniziert. Während des Betriebs der Pumpeneinheit kann das magnetische Spülventil, beispielsweise in vorbestimmten Intervallen, von dem Steuerungsmittel kurzzeitig, beispielsweise 100 Millisekunden, geöffnet werden. Da die Belüftungs- oder Spülleitung über den Wundraum in Fluidkommunikation mit der Drainageleitung steht, werden nach einer kurzen Ventilöffnung Luftstöße in der Drainageleitung erzeugt. Derartige Luftstöße in der Drainageleitung können gegebenenfalls vorhandene Verklumpungen lösen und auf diese Weise das Risiko vermindern, dass die Drainageleitung im Unterdruckbetrieb durch Wundflüssigkeit und Gewebereste verschlossen werden kann. Solange das Belüftungs- oder Spülventil gegenüber der Umgebung geschlossen ist, kann im Inneren der Belüftungs- oder Spülleitung ein im Wesentlichen dem Wundraum entsprechender Unterdruck gemessen werden. Es ist daher möglich, Drucksensormittel im Inneren einer solchen Belüftungs- oder Spülleitung oder in einem von dieser Leitung abzweigenden Leitungsstück anzubringen. Eine derartige Anordnung des Drucksensormittels ist besonders vorteilhaft, weil die Belüftungs- oder Spülleitung stets nur mit Luft gespült wird und daher nicht verstopfen kann. Dies gewährleistet stets eine genaue Ermittlung des im Wundraum angelegten Unterdrucks durch das Drucksensormittel.

Die Öffnungsimpulse erhält das Ventil normalerweise über eine Kommunikationsverbindung mit dem Steuerungsmittel. Denkbar ist es auch, das Belüftungsventil mit einem autonom arbeitenden Öffnungsmechanismus zu versehen.
Wesentlich für alle erfindungsgemäßen Ausführungsformen ist es, dass das Drucksensormittel zum Zeitpunkt der Bestimmung des Unterdrucks in Fluidkommunikation mit dem Wundraum steht. Eine Fluidkommunikation mit dem Wundraum kann auch indirekt hergestellt werden, beispielsweise über die Drainageleitung oder über den Drainagebehälter.

Erfindungsgemäß umfasst die Wundtherapievorrichtung ein Mittel zum Zuführen eines Instillfluids aus dem Instillbehälter in den Wundraum. Bei dem Mittel zum Zuführen eines Instillfluids aus dem Instillbehälter in den Wundraum kann es sich bei einer einfach gestalteten Ausführungsform der Erfindung beispielsweise um einen Infusionsständer handeln. Bei dieser Ausführungsform wird der Instillbehälter so angebracht, dass das Instillfluid durch die Schwerkraft in die Wunde fließen kann.

Weiterhin ist es auch möglich, dass das Instillfluid alleine durch die Saugkraft des an der Wunde angelegten Unterdrucks in den Wundraum gesaugt wird.

Gemäß einer bevorzugten Ausführungsform der Erfindung handelt es sich bei dem Mittel zum Zuführen eines Instillfluids aus dem Instillbehälter in den Wundraum um eine Pumpeneinheit, wobei die Pumpeneinheit fluidleitend mit dem Instillbehälter verbunden ist. Die Pumpeneinheit ist bei Erreichen eines voreingestellten unteren Unterdruck-Schwellenwertes im Wundraum abschaltbar, so dass die weitere Zufuhr des Instillfluids unterbrochen wird. Die Abschaltung erfolgt insbesondere durch ein von dem Steuerungsmittel bei Erreichen eines voreingestellten unteren Unterdruck-Schwellenwertes im Wundraum ausgegebenes Signal.

Vorzugsweise handelt es sich bei dem Mittel zum Zuführen eines Instillfluids aus dem Instillbehälter in den Wundraum um eine zusätzlich zur ersten Pumpeneinheit vorhandene zweite Pumpeneinheit (Fluid-Förderpumpe). Die Fluid-Förderpumpe weist eine Kommunikationsverbindung mit dem Steuerungsmittel auf. Bei Erreichen eines voreingestellten unteren Unterdruck-Schwellenwertes im Wundraum gibt das Steuerungsmittel über die Kommunikationsverbindung ein Signal zum Abschalten der zweiten Pumpeneinheit aus, so dass die weitere Zufuhr des Instillfluids unterbrochen wird.

Gemäß einer alternativen Ausführungsform der Erfindung umfasst die Instilltherapievorrichtung ein Ventil bzw. Absperrventil, welches die fluidleitende Verbindung zwischen dem Instillbehälter und dem Unterdruckverband schließen kann. Zwischen dem Ventil und dem Steuerungsmittel ist eine Kommunikationsverbindung vorhanden, so dass das Ventil bei Erreichen eines unteren Unterdruck-Schwellenwertes durch das Steuerungsmittel schließbar ist. Durch das Schließen des Ventils wird die weitere Zufuhr des Instillfluids unterbrochen.

Es kann auch vorgesehen sein, dass die Vorrichtung gleichzeitig ein Ventil und eine zweite Pumpeneinheit umfasst. Solchenfalls kann durch das Schließen des Ventils und durch das gleichzeitige Abschalten der zweiten Pumpeneinheit noch besser sichergestellt werden, dass bei Erreichen eines voreingestellten unteren Unterdruck-Schwellenwertes im Wundraum kein weiteres Instillfluid in die Wunde eingeleitet wird.

Bereits bei einem Unterdruck von weniger als 3999,66 Pa (30 mm Hg) (Druckdifferenz zum Umgebungsdruck) nähert sich der Unterdruck in der Wunde dem Umgebungsdruck an. Somit ist gewährleistet, dass eine optimale Menge an Instillfluid in die Wunde eingeleitet wurde. Gemäß einer bevorzugten Ausführungsform der Erfindung liegt der untere Schwellenwert für den Unterdruck zwischen 0 Pa (0 mm Hg) und 3999,66 Pa (30 mm Hg), bevorzugt zwischen 666,61 Pa (5 mm Hg) und 3333,05 Pa (25 mm Hg) und noch mehr bevorzugt zwischen 1333,22 Pa (10 mm Hg) und 2666,44 Pa (20 mm Hg).

Gemäß einer bevorzugten Ausführungsform der Erfindung ist die Instilltherapievorrichtung tragbar. Tragbar bedeutet, dass die Instilltherapievorrichtung aufgrund ihren Ausgestaltung und ihres Gewichtes für einen Patienten mitnehmbar ist. Weiterhin muss eine tragbare Instilltherapievorrichtung über eine geeignete autonome Energieversorgung verfügen, beispielsweise über eine aufladbare Batterie. Eine tragbare Instilltherapievorrichtung fördert die Mobilität des Patienten.

Weiter erweist es sich als vorteilhaft, wenn die Wundtherapievorrichtung ein Temperaturregulierungselement für die Regulierung der Temperatur des Instillfluids aufweist. Das Temperaturregulierungselement umfasst einen Temperatursensor. Das Temperaturregulierungselement ist bevorzugt im Inneren des Instillbehälters oder an dessen Außenseite angeordnet. Alternativ dazu könnte das Temperaturregulierungselement auch an der Instillleitung angeordnet sein und nur die Temperatur des in die Wunde eingeleiteten Instillfluids verändern. Dies kann beispielsweise bei der Applikation von Instilllösungen, welche ein wärmeempfindliches Medikament enthalten, vorteilhaft sein. Eine Temperierung des Instillfluids, beispielsweise auf 30°C bis 37°C, kann für die die Wundheilung förderlich sein. Insbesondere kann eine gegenüber dem Stand der Technik deutlich verbesserte Instilltherapie durchgeführt werden, indem eine erfindungsgemäß exakt auf das Volumen des Wundraums abgestimmte Menge eines temperierten Instillfluids verabreicht wird.

Vorzugsweise umfasst das Instillfluid eine Flüssigkeit. Flüssigkeiten sind insbesondere dafür geeignet, nekrotisches Gewebe zu lösen, die Wunde zu reinigen und die Wundflüssigkeit zu verdünnen. Auf diese Weise tragen Flüssigkeiten zum effizienten Debridement der Wunde bei. Gemäß einer weiteren bevorzugten Ausführungsform umfasst das Instillfluid mindestens eine Puffersubstanz. Durch die Applikation von Flüssigkeiten, welche Puffersubstanzen umfassen, kann der pH-Wert der Wunde beeinflusst und so ein für die Wundheilung besonders günstiges Wundmillieu hergestellt werden. Bei der Applikation einer Instillflüssigkeit, welche eine oder mehrere Puffersubstanzen umfasst, kommt es besonders auf eine geeignete Dosierung des zugeführten Flüssigkeitsvolumens an, da andernfalls die gewünschte, den pH-Wert der Wunde beeinflussende Wirkung möglicherweise nicht erreicht wird.

Optional kann die Flüssigkeit Wirkstoffe in gelöster Form oder als Kolloide enthalten. Die Wirkstoffe können dabei an Trägersubstanzen gebunden sein. Geeignete Wirkstoffe mit wundheilungsfördernden Eigenschaften sind beispielsweise Antiseptika, Antibiotika, Wachstumsfaktoren, Gerinnungsfaktoren oder die Gewebeheilung unterstützende Nährstoffe.

Vorzugsweise umfasst die Flüssigkeit Wasser oder eine Elektrolytlösung, insbesondere Ringerlösung. Eine Elektrolytlösung, insbesondere eine isotone Elektrolytlösung, kann in vorteilhafter Weise als Instilllösung eingesetzt werden, weil sie eine den Körperflüssigkeiten ähnliche Salzkonzentration aufweist und daher osmotisch inaktiv ist. Eine osmotisch inaktive Instillflüssigkeit ist besonders gewebeschonend und unterstützt die Wundheilung. Gemäß einer besonders bevorzugten Ausführungsform umfasst die Spülflüssigkeit Ringerlösung. Die vorteilhafte Wirkung der Ringerlösung bei der Unterdruckbehandlung von Wunden ist in der so genannten Reinigungsphase der Wundheilung besonders ausgeprägt. Unter einer Ringerlösung wird eine annähernd zum Blut iso-osmotische synthetische Lösung verstanden, welche Natriumchlorid, Kaliumchlorid und Calciumchlorid umfasst. Ringerlösung lässt sich beispielsweise herstellen, indem 8,6 g Natriumchlorid, 0,30 g Kaliumchlorid und 0,33 g Calciumchlorid (Dihydrat) in einem Liter destillierten Wasser aufgelöst werden.

Nach einem weiteren Erfindungsgedanken kann es sich als vorteilhaft erweisen, wenn bei der Instilltherapie eine Instillflüssigkeit mit hypertonen Eigenschaften verwendet wird. Eine Instillflüssigkeit mit hypertonen Eigenschaften weist im Vergleich zu der Salzkonzentration der Körperflüssigkeiten eine höhere Salzkonzentration auf. Eine Instillflüssigkeit mit hypertonen Eigenschaften ist osmotisch aktiv und unterstützt die Abgabe von Gewebeflüssigkeit in den Wundraum. Die Verwendung einer Instillflüssigkeit mit hypertonen Eigenschaften kann besonders geeignet für die Therapie von Wunden mit großen Anteilen an nekrotischem Gewebe sein.

Nach einem unabhängigen Erfindungsgedanken erweist es sich als vorteilhaft, wenn das Instillfluid ein Gas umfasst.

Weiter erweist es sich hierbei als vorteilhaft, wenn das Gas ausgewählt wird aus den Gasen Luft, mit Sauerstoff angereicherte Luft oder Stickstoff. Ein Vorteil von Luft ist es, dass die Luft überall verfügbar ist und nach entsprechender Entkeimung sofort eingesetzt werden kann. Mit Sauerstoff angereicherte Luft ist vorteilhaft, weil sie das Gewebe im Wundraum zum Wachsen anregen können. Unter einer mit Sauerstoff angereicherten Luft wird hierbei eine Luft verstanden, die einen gegenüber der Umgebungsluft erhöhten Sauerstoffanteil aufweist, also einen Sauerstoffanteil von mehr als 21 %. Die Verwendung von Stickstoff ist vorteilhaft, weil Stickstoff eine sehr hohe Haut- und Gewebeverträglichkeit aufweist. Nach einem weiteren Erfindungsgedanken wird vorgeschlagen, dass das Gas ein oder mehrere zerstäubte Wirkstoffe in Aerosolform umfasst. Es kann auch vorgesehen sein, dass das Gas Geruch-absorbierende Stoffe umfasst.

### Anwendung der erfindungsgemäßen Vorrichtung

Auf die zu behandelnde Wunde wird ein auf die Größe der Wunde zurecht geschnittener Polyurethanschaum aufgebracht. Der Polyurethanschaum befindet sich in direktem Kontakt mit der Wundoberfläche. Der Polyurethanschaum und die unmittelbare Umgebung der Wunde werden mit einer klebenden, fluiddichten Abdeckfolie abgedeckt, so dass zwischen Abdeckfolie und Wundgrund ein Wundraum gebildet wird. Die Folie haftet auf der die Wunde umgebenden intakten Haut des Patienten und verschließt den Wundraum gasdicht. Auf der Folie wird ein Unterdruckanschlussmittel (Port) befestigt, mittels dem Fluidverbindungen zwischen dem Wundraum und der Instillleitung, zwischen dem Wundraum und der Drainageleitung und zwischen dem Wundraum und der - falls vorhanden - Spülleitung hergestellt wird.

Durch Ingangsetzen der Unterdruckpumpe (erste Pumpeneinheit) wird ein Unterdruck im Wundraum, also in dem durch Abdeckfolie und Wundgrund gebildeten Zwischenraum, erzeugt. Der im Wundraum vorhandene Unterdruck wird durch ein Drucksensormittel, welches mit dem Steuerungsmittel kommuniziert, erfasst und kontinuierlich überwacht. Sobald das Drucksensormittel einen Unterdruck oberhalb eines voreingestellten oberen Schwellenwertes registriert, wird die erste Pumpeneinheit abgeschaltet. Der obere Schwellenwertes kann gegebenenfalls vor der Wundtherapie durch einen Benutzer manuell voreingestellt werden, beispielsweise über eine Eingabeeinheit. Eine geeignete Eingabeeinheit ist beispielsweise eine Tastatur oder ein Touchscreen. Als oberer Schwellenwert könnte beispielsweise ein Unterdruck zwischen 9999,15 Pa (75 mm Hg) und 19998,3 Pa (150 mm Hg) dienen. Vorzugsweise liegt der obere Schwellenwert im Bereich zwischen 13332,2 Pa (100 mm Hg) und 17331,86 Pa (130 mm Hg), insbesondere beträgt er 13332,2 Pa (100 mm Hg), 16665,25 Pa (125 mm Hg) oder 17331,86 Pa (130 mm Hg).

Nach der Inaktivierung der Unterdruckpumpe wird das Mittel zum Zuführen des Instillfluids aktiviert, so dass Instillfluid in den Wundraum gelangen kann. Vorzugsweise handelt es sich bei dem Mittel zum Zuführen des Instillfluids um eine zweite Pumpeneinheit (Fluid-Förderpumpe), welche unter Kontrolle des Steuerungsmittels steht.
Durch die Zufuhr von Instillfluid in die Wunde verringert sich der Unterdruck im Wundraum. Unter Verringerung des Luftdrucks wird hierbei verstanden, dass sich der Unterdruck dem Umgebungsluftdruck annähert. Der abnehmende Unterdruck wird durch das Drucksensormittel registriert. Sobald der Unterdruck einen unteren Schwellenwert erreicht, wird die zweite Pumpeneinheit durch das Steuerungsmittel abgeschaltet. Der untere Schwellenwertes kann wie der obere Schwellenwert gegebenenfalls vor der Wundtherapie durch einen Benutzer manuell voreingestellt werden, beispielsweise über eine Eingabeeinheit. Als unterer Schwellenwert kann beispielsweise ein Unterdruck zwischen 0 Pa (0 mm Hg) und 4666,27 Pa (35 mm Hg), insbesondere ein Unterdruck von 0 Pa (0 mm Hg), 666,61 Pa (5 mm Hg), 1333,22 Pa (10 mm Hg), 1999,83 Pa (15 mm Hg), 2666,44 Pa (20 mm Hg), 3333,05 Pa (25 mm Hg) oder 3999,66 Pa (30mm Hg) gewählt werden.

Das Instillfluid verbleibt anschließend für einen voreingestellten Zeitraum, beispielsweise 30 Sekunden bis 10 Minuten, in der Wunde und kann währenddessen auf den Wundgrund einwirken.

Nach Ende des voreingestellten Zeitraums kann die erste Pumpeneinheit wieder eingeschaltet werden und das Instillfluid zusammen mit der Wundflüssigkeit über die Drainageleitung abgesaugt werden, bis erneut der obere Schwellenwert für den Unterdruck im Wundraum erreicht ist. Derartige Spül- und Saugzyklen können bis zum Ende der Instilltherapie wiederholt werden.

### Figurenteil

Nachstehend werden Ausführungsformen erfindungsgemäßer Wundtherapievorrichtungen anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die Erfindung auch Kombinationen der Einzelmerkmale der alternativen Formen. Es zeigen:

### Kurzbeschreibung der Figuren:

Fig. 1) Eine erste Ausführungsform der erfindungsgemäßen Wundtherapievorrichtung (schematische Darstellung). Die dargestellte Wundtherapievorrichtung umfasst eine erste und eine zweite Pumpeneinheit.
Fig. 2) Eine weitere Ausführungsform der erfindungsgemäßen Instilltherapievorrichtung (schematische Darstellung). Die dargestellte Wundtherapievorrichtung umfasst eine erste Pumpeneinheit.
Fig. 3) Eine weitere Ausführungsform der erfindungsgemäßen Wundtherapievorrichtung, welche einen im Fluidweg zwischen dem Drainagebehälter und der ersten Pumpeneinheit angeordneten Druckmesser aufweist (schematische Darstellung).
Fig. 4) Eine weitere Ausführungsform der erfindungsgemäßen Wundtherapievorrichtung, welche eine zusätzliche Druckmessleitung aufweist (schematische Darstellung).

### Figurenlegende

1 Drainagebehälter
2 Drucksensormittel
3 Drainageleitung
4 Erste Pumpeneinheit (Unterdruckpumpe)
5 Steuerungsmittel
7 Instillbehälter
8 Instillleitung
9 Zweite Pumpeneinheit (Fluid-Förderpumpe)
10 Display
11 Eingabeeinheit
12 Temperaturregulierungselement
13 Temperatursensor
15 Unterdruckverband
16 Unterdruckanschlussstück (Port)
17 Abdeckfolie
20, 40, 50, 60 Wundtherapievorrichtung
21 Spülleitung
22 Spülventil
23 Bakterienfilter
41 Spül- oder Belüftungsventil
61 Druckmessleitung

### Ausführliche Beschreibung der Figuren:

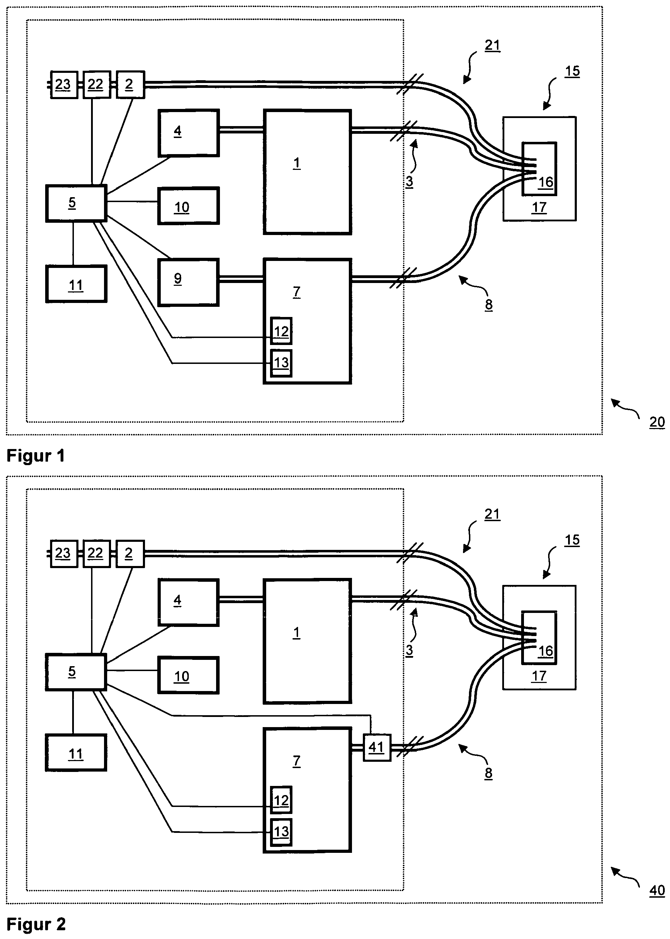

Figur 1 zeigt eine erfindungsgemäße Wundtherapievorrichtung 20. Die Wundtherapievorrichtung 20 für die Behandlung von Wunden umfasst einen Drainagebehälter 1 zur Sammlung von Flüssigkeit. Der Behälter 1 besteht aus einem Kunststoff und weist ein Volumen von ca. 800 ml auf. Weiterhin umfasst die Wundtherapievorrichtung eine Drainageleitung 3, welche den ersten Behälter 1 fluidleitend mit dem Unterdruckverband 15 und dem Wundraum verbindet. Die Drainageleitung 3 umfasst einen biegsamen Kunststoffschlauch, beispielsweise aus Silikon, mit einer inneren offenen Querschnittsfläche von beispielsweise 10 bis 200 mm². Der Unterdruckverband 15 umfasst einen offenzelligen Polyurethanschaum als Wundauflage (nicht dargestellt) und eine Abdeckfolie 17, wobei der Polyurethanschaum unter der Abdeckfolie 17 auf den Wundgrund aufgebracht wird. Bei der gezeigten Ausführungsform sind die Drainageleitung 3, die Instillleitung 8 und die Spülleitung 21 mittels eines einzigen Ports 16 mit der Abdeckfolie 17 verbunden.

Die Spülleitung 21 steht in Fluidkommunikation mit dem Drucksensormittel 2. Das Drucksensormittel 2 ist bei der in Figur 1 gezeigten Ausführungsform im Inneren der Spülleitung 21 angeordnet. Die Spülleitung 21 weist an ihrem wundabseitig orientierten Endbereich das Drucksensormittel 2, das Spülventil 22 und den Bakterienfilter 23 auf. Die am Drucksensormittel 2 ermittelten Druckmessdaten werden über eine Kommunikationsverbindung an das Steuerungsmittel 5 weitergegeben. Das Drucksensormittel 2 ist im Inneren der Spülleitung 21 oder alternativ in einem von der Spülleitung 21 abzweigenden Leitungsabschnitt (nicht dargestellt) angebracht und gewährleistet somit eine Überwachung des Unterdrucks im Wundraum, solange das Spülventil 22 geschlossen ist.

Die erste Pumpeneinheit (Unterdruckpumpe) 4 ist fluidleitend mit dem ersten Behälter 1 verbunden und wird durch das Steuerungsmittel 5 kontrolliert. Die Unterdruckpumpe 4 umfasst eine Membranpumpe, die direkt über das Steuerungsmittel 5 ansteuerbar ist. Bei dem Steuerungsmittel 5 handelt es sich um ein elektronisches Bauelement, vorzugsweise um ein programmierbares elektronisches Bauelement, insbesondere um einen programmierbaren Mikroprozessor. An das elektronische Bauelement ist vorzugsweise eine geeignete Eingabeeinheit 11 angeschlossen, beispielsweise eine Tastatur oder ein Touchpad, um dem Benutzer das Eingeben von Programmen für eine Unterdruck- und Instilltherapie zu ermöglichen. Das Steuerungsmittel 5 verfügt vorzugsweise über ein Anzeigemittel in Form eines Displays 10. Zwischen dem Steuerungsmittel 5 und dem Drucksensormittel 2 ist eine Kommunikationsverbindung, beispielsweise in Form eines Kabels für die serielle Datenübertragung, vorhanden.

Die Wundtherapievorrichtung umfasst einen zweiten Behälter (Instillbehälter) 7 zur Bereitstellung eines Instillfluids und eine Instillleitung 8, welche den Instillbehälter 7 fluidleitend mit dem Wundraum verbindet. Der Instillbehälter 7 ist ebenso wie der erste Behälter 1 ein Kunststoffbehälter mit einem Volumen von ca. 800 ml. Optional könnte der Instillbehälter 7 auch als flexibler, flüssigkeitsundurchlässiger Beutel ausgebildet sein. Als Instillfluid wird vorzugsweise Ringerlösung verwendet.

Bei der in Figur 1 gezeigten bevorzugten Ausführungsform der Erfindung umfasst das Mittel zum Zuführen eines Instillfluids aus dem Instillbehälter (7) in den Wundraum eine zweite Pumpeneinheit (Fluid-Förderpumpe) 9, welche durch das Steuerungsmittel 5 kontrolliert wird. Hierzu ist eine Kommunikationsverbindung zwischen der Fluid-Förderpumpe 9 und dem Steuerungsmittel 5 vorgesehen. Die Fluid-Förderpumpe 9 ist fluidleitend mit dem Instillbehälter 7 verbunden und bewirkt die Zufuhr von Instillfluid in den Wundraum. Die Fluid-Förderpumpe 9 kann wie die erste Pumpeneinheit (Unterdruckpumpe) 4 eine Membranpumpe umfassen.

In dem Instillbehälter 7 ist optional ein Temperaturregulierungselement 12 mit einem Temperatursensor 13 vorgesehen, welches die Temperatur des Instillfluids verändern kann. Der Temperatursensor 13 ist über ein Kabel, insbesondere über ein Kabel für die serielle Datenübertragung, mit dem Steuerungsmittel 5 verbunden.

Die in Figur 1 dargestellte Ausführungsform ist tragbar und für den mobilen Einsatz vorgesehen. Alle elektrischen beziehungsweise elektronischen Bauelemente werden durch eine aufladbare Batterie (nicht dargestellt) mit Energie versorgt.

Figur 2 zeigt eine alternative Ausführungsform der erfindungsgemäßen Wundtherapievorrichtung 40. Die Wundtherapievorrichtung 40 für die Behandlung von Wunden unterscheidet sich von der in Figur 1 dargestellten Vorrichtung 20 dahingehend, dass ein Ventil 41 zwischen dem Instillbehälter 7 und der Instillleitung 8 vorgesehen ist. Das Ventil 41 ist vorgesehen, um bei Erreichen eines voreingestellten unteren Unterdruck-Schwellenwertes im Wundraum (beispielsweise 1333,22 Pa (10 mm Hg)) die fluidleitende Instillleitung 8 zwischen dem zweiten Behälter 7 und der Unterdruckwundauflage 15 zu schließen. Im Unterschied zu der Wundtherapievorrichtung 20 ist keine Fluid-Förderpumpe vorhanden. Stattdessen ist ein alternatives Mittel zum Zuführen eines Instillfluides aus dem Behälter 7 vorgesehen. Im einfachsten Fall wird das Instillfluid dem Wundraum durch die Saugkraft des an der Wunde angelegten Unterdrucks zugeführt. Gemäß der in Figur 2 gezeigten Ausführungsform ist eine Kommunikationsverbindung zwischen dem Ventil 41 und dem Steuerungsmittel 5 vorhanden.

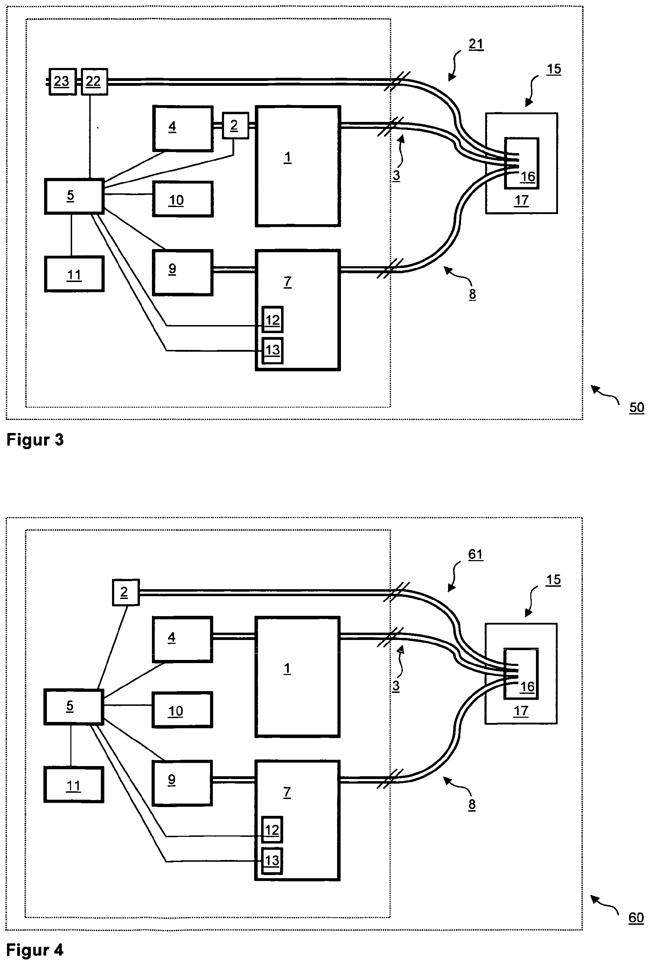

Figur 3 zeigt eine weitere alternative Ausführungsform der erfindungsgemäßen Wundtherapievorrichtung 50. Die Wundtherapievorrichtung 50 unterscheidet sich von der in Figur 1 dargestellten Vorrichtung 20 durch die Anordnung des Drucksensormittels 2 im Fluidweg zwischen Drainagebehälter 1 und erster Pumpeneinheit 4.

Bei der in Figur 4 gezeigten Ausführungsform umfasst die Wundtherapievorrichtung 60 eine zusätzliche Druckmessleitung 61 zwischen Wundverband 15 und Drucksensormittel 2. Die zusätzliche Druckmessleitung 61 stellt eine Fluidverbindung zwischen Wundraum und Drucksensormittel 2 her. Proximal endet die zusätzliche Druckmessleitung 61 deshalb am Unterdruckverband 15, wobei eine FluidVerbindung mit dem Wundraum beispielsweise mittels eines Unterdruckanschlussstückes erfolgen kann. Am distalen Ende der Druckmessleitung 61 befindet sich das Drucksensormittel 2, welches vorzugsweise innerhalb des Gehäuses des Therapiegerätes angeordnet ist. Da das Drucksensormittel 2 somit über die Druckmessleitung 61 in Fluidkommunikation mit dem Wundraum steht, kann der Unterdruck im Wundraum durch ein am Therapiegerät oder im Therapiegerät angebrachtes Drucksensormittel überwacht werden.

## Patentansprüche

1. Wundtherapievorrichtung (20, 40, 50, 60) für die Behandlung von Wunden mittels Unterdruck und Fluidspülung, umfassend
i. einen Unterdruckverband (15) umfassend eine fluiddichte Abdeckung, so dass ein Wundraum zwischen der Abdeckung und dem Wundgrund gebildet werden kann
ii. ein Drucksensormittel (2), welches Druckmesswerte erzeugen kann, wobei das Drucksensormittel (2) in Fluidkommunikation mit dem Wundraum steht
iii. einen Drainagebehälter (1) zur Sammlung von Flüssigkeit
iv. eine Drainageleitung (3), mittels derer Flüssigkeit aus dem Wundraum in den Drainagebehälter (1) geleitet werden kann
v. eine erste Pumpeneinheit (4) zum Absaugen von Flüssigkeit aus dem Wundraum über die Drainageleitung (3), wobei die erste Pumpeneinheit (4) fluidleitend mit dem Drainagebehälter (1) verbunden ist
vi. einen Instillbehälter (7) zur Bereitstellung eines Instillfluids
vii. eine Instillleitung (8), mittels derer Instillfluid aus dem Instillbehälter (7) in den Wundraum zugeführt werden kann
viii. ein Mittel zum Zuführen eines Instillfluids aus dem Instillbehälter (7) in den Wundraum
ix. ein Steuerungsmittel (5), wobei das Steuerungsmittel (5) Druckmesswerte vom Drucksensormittel (2) empfangen kann
**dadurch gekennzeichnet,**
x. dass das Steuerungsmittel bei Erreichen eines voreingestellten oberen Unterdruck-Schwellenwertes im Wundraum ein Signal zum Abschalten der ersten Pumpeneinheit (4) ausgeben kann, und
xi. dass das Steuerungsmittel bei Erreichen eines voreingestellten unteren Unterdruck-Schwellenwertes im Wundraum ein Signal zum Unterbrechen der Zufuhr des Instillfluids ausgeben kann.

2. Wundtherapievorrichtung (20, 40, 50, 60) nach Anspruch 1, weiterhin umfassend ein Mittel zur Bestimmung des Volumens an Instillfluid, welches in den Wundraum eingebracht wird.

3. Wundtherapievorrichtung (20, 50, 60) nach Anspruch 1 oder 2, wobei es sich bei dem Mittel zum Zuführen eines Instillfluids aus dem Instillbehälter (7) in den Wundraum um eine zweite Pumpeneinheit (9) handelt, welche mit dem Instillbehälter (7) fluidleitend verbunden ist.

4. Wundtherapievorrichtung (40) nach einem der vorhergehenden Ansprüche, weiterhin umfassend ein Ventil (41), welches die fluidleitende Verbindung zwischen dem Instillbehälter (7) und dem Wundraum schließen kann, wobei das vom Steuerungsmittel ausgegebene Signal zum Unterbrechen der Zufuhr des Instillfluids das Schließen des Ventils (41) bewirkt.

5. Wundtherapievorrichtung (20, 40, 50, 60) nach einem der vorhergehenden Ansprüche, wobei das Drucksensormittel (2) im Wundraum angeordnet ist.

6. Wundtherapievorrichtung (20, 40, 50, 60) nach einem der vorhergehenden Ansprüche, wobei das Drucksensormittel (2) in Fluidkommunikation mit der Drainageleitung (3) und/oder dem Drainagebehälter (1) steht.

7. Wundtherapievorrichtung (20, 40, 50, 60) nach einem der vorhergehenden Ansprüche, wobei der Drainagebehälter (1) im Fluidweg zwischen der ersten Pumpeneinheit (4) und der Drainageleitung (3) angeordnet ist.

8. Wundtherapievorrichtung (50) nach einem der vorhergehenden Ansprüche, wobei das Drucksensormittel (2) im Fluidweg zwischen der ersten Pumpeneinheit (4) und dem Drainagebehälter (1) angeordnet ist.

9. Wundtherapievorrichtung (60) nach einem der vorhergehenden Ansprüche, wobei die Wundtherapievorrichtung eine separate Druckmessleitung (61) aufweist und wobei die Druckmessleitung (61) in Fluidkommunikation mit dem Unterdruckverband (15) und mit dem Drucksensormittel (2) steht.

10. Wundtherapievorrichtung (20, 40, 50) nach einem der vorhergehenden Ansprüche, wobei die Wundtherapievorrichtung eine Belüftungsleitung oder eine Spülleitung (21) aufweist und wobei die Belüftungsleitung oder Spülleitung (21) in Fluidkommunikation mit dem Drucksensormittel (2) steht.

11. Wundtherapievorrichtung (20, 40, 50, 60) nach einem der vorhergehenden Ansprüche, wobei der untere Unterdruck-Schwellenwert im Wundraum zwischen 0 Pa (0 mm Hg) und 3999,66 Pa (30 mm Hg), bevorzugt zwischen 666,61 Pa (5 mm Hg) und 3333,05 Pa (25 mm Hg) und noch mehr bevorzugt zwischen 1333,22 Pa (10 mm Hg) und 2666,44 Pa (20 mm Hg) liegt.

12. Wundtherapievorrichtung (20, 40, 50, 60) nach einem der vorhergehenden Ansprüche, wobei der obere Unterdruck-Schwellenwert im Wundraum zwischen 13332,2 Pa (100 mm Hg) und 17331,86 Pa (130 mm Hg), insbesondere zwischen 15998,64 Pa (120 mm Hg) und 17331,86 Pa (130 mm Hg) liegt.

13. Wundtherapievorrichtung (20, 40, 50, 60) nach einem der vorhergehenden Ansprüche, wobei die Wundtherapievorrichtung ortsgebunden oder tragbar ist.

14. Wundtherapievorrichtung (20, 40, 50, 60) nach einem der vorhergehenden Ansprüche, wobei die Wundtherapievorrichtung ein Temperaturregulierungselement (12) für die Regulierung der Temperatur des Instillfluids aufweist.

15. Wundtherapievorrichtung (20, 40, 50, 60) nach Anspruch 14, wobei das Temperaturregulierungselement (12) ein Durchströmungsheizelement umfasst.

16. Wundtherapievorrichtung (20, 40, 50, 60) nach einem der vorhergehenden Ansprüche, wobei das Instillfluid eine Flüssigkeit umfasst.

17. Wundtherapievorrichtung (20, 40, 50, 60) nach Anspruch 16, wobei die Flüssigkeit ausgewählt ist aus Wasser, Elektrolytlösung oder Ringerlösung,

18. Wundtherapievorrichtung (20, 40, 50, 60) nach einem der vorhergehenden Ansprüche, wobei das Instillfluid ein Gas umfasst.

19. Wundtherapievorrichtung (20, 40, 50, 60) nach Anspruch 18, wobei das Gas ausgewählt ist aus den Gasen Luft, mit Sauerstoff angereicherter Luft oder Stickstoff.

## Claims

1. Wound therapy device (20, 40, 50, 60) for the treatment of wounds by means of negative pressure and fluid irrigation, comprising
i. a negative-pressure dressing (15) with a fluid-tight cover, such that a wound space can be formed between the cover and the wound base,
ii. a pressure sensor means (2), which can generate pressure measurement values, wherein the pressure sensor means (2) is in fluidic communication with the wound space,
iii. a drainage container (1) for collecting liquid,
iv. a drainage line (3) by means of which liquid can be conveyed from the wound space into the drainage container (1),
v. a first pump unit (4) for aspirating liquid from the wound space via the drainage line (3), wherein the first pump unit (4) is connected in a fluid-conducting manner to the drainage container (1),
vi. an instillation container (7) for making available an instillation fluid,
vii. an instillation line (8) by means of which instillation fluid can be delivered from the instillation container (7) into the wound space,
viii. a means for delivering an instillation fluid from the instillation container (7) into the wound space,
ix. a control means (5), wherein the control means (5) can receive pressure measurement values from the pressure sensor means (2),
**characterized in that**
x. when a predefined upper negative-pressure threshold value is reached in the wound space, the control means can output a signal to switch off the first pump unit (4), and
xi. when a predefined lower negative-pressure threshold value is reached in the wound space, the control means can output a signal to interrupt the delivery of the instillation fluid.

2. Wound therapy device (20, 40, 50, 60) according to Claim 1, further comprising a means for determining the volume of instillation fluid that is introduced into the wound space.

3. Wound therapy device (20, 50, 60) according to Claim 1 or 2, wherein the means for delivering an instillation fluid from the instillation container (7) into the wound space is a second pump unit (9), which is connected in a fluid-conducting manner to the instillation container (7).

4. Wound therapy device (40) according to one of the preceding claims, further comprising a valve (41) which can close the fluid-conducting connection between the instillation container (7) and the wound space, wherein the signal output by the control means to interrupt the delivery of the instillation fluid causes the closure of the valve (41).

5. Wound therapy device (20, 40, 50, 60) according to one of the preceding claims, wherein the pressure sensor means (2) is arranged in the wound space.

6. Wound therapy device (20, 40, 50, 60) according to one of the preceding claims, wherein the pressure sensor means (2) is in fluidic communication with the drainage line (3) and/or the drainage container (1).

7. Wound therapy device (20, 40, 50, 60) according to one of the preceding claims, wherein the drainage container (1) is arranged in the fluid path between the first pump unit (4) and the drainage line (3).

8. Wound therapy device (50) according to one of the preceding claims, wherein the pressure sensor means (2) is arranged in the fluid path between the first pump unit (4) and the drainage container (1).

9. Wound therapy device (60) according to one of the preceding claims, wherein the wound therapy device has a separate pressure measurement line (61), and wherein the pressure measurement line (61) is in fluidic communication with the negative-pressure dressing (15) and with the pressure sensor means (2).

10. Wound therapy device (20, 40, 50) according to one of the preceding claims, wherein the wound therapy device has a ventilation line or an irrigation line (21), and wherein the ventilation line or irrigation line (21) is in fluidic communication with the pressure sensor means (2).

11. Wound therapy device (20, 40, 50, 60) according to one of the preceding claims, wherein the lower negative-pressure threshold value in the wound space is between 0 Pa (0 mmHg) and 3999.66 Pa (30 mmHg), preferably between 666.61 Pa (5 mmHg) and 3333.05 Pa (25 mmHg), and still more preferably between 1333.22 Pa (10 mmHg) and 2666.44 Pa (20 mmHg).

12. Wound therapy device (20, 40, 50, 60) according to one of the preceding claims, wherein the upper negative-pressure threshold value in the wound space is between 13332.2 Pa (100 mmHg) and 17331.86 Pa (130 mmHg), in particular between 15998.64 Pa (120 mmHg) and 17331.86 Pa (130 mmHg).

13. Wound therapy device (20, 40, 50, 60) according to one of the preceding claims, wherein the wound therapy device is stationary or portable.

14. Wound therapy device (20, 40, 50, 60) according to one of the preceding claims, wherein the wound therapy device has a temperature-regulating element (12) for regulating the temperature of the instillation fluid.

15. Wound therapy device (20, 40, 50, 60) according to claim 14, wherein the temperature-regulating element (12) comprises a through-flow heating element.

16. Wound therapy device (20, 40, 50, 60) according to one of the preceding claims, wherein the instillation fluid comprises a liquid.

17. Wound therapy device (20, 40, 50, 60) according to claim 16, wherein the liquid is chosen from water, electrolyte solution or Ringer's solution.

18. Wound therapy device (20, 40, 50, 60) according to one of the preceding claims, wherein the instillation fluid comprises a gas.

19. Wound therapy device (20, 40, 50, 60) according to claim 18, wherein the gas is chosen from the gases air, oxygen-enriched air, or nitrogen.

## Revendications

1. Dispositif pour la thérapie des plaies (20, 40, 50, 60) pour le traitement de plaies au moyen d'une pression négative et d'un rinçage par un fluide, comprenant
i. un pansement à pression négative (15) comprenant une couverture étanche au fluide, de telle manière qu'une cavité de plaie puisse être formée entre la couverture et le fond de la plaie,
ii. un moyen de capteur de pression (2), qui peut produire des valeurs de mesure de pression, dans lequel le moyen de capteur de pression (2) est en communication fluidique avec la cavité de plaie,
iii. un réservoir de drainage (1) pour la collecte de liquide,
iv. une conduite de drainage (3), au moyen de laquelle du liquide peut être conduit hors de la cavité de plaie dans le réservoir de drainage (1),
v. une première unité de pompe (4) pour aspirer du liquide hors de la cavité de plaie par la conduite de drainage (3), dans lequel la première unité de pompe (4) est reliée en conduite de fluide au réservoir de drainage (1),
vi. un réservoir d'instillation (7) pour la fourniture d'un fluide à instiller,
vii. une conduite d'instillation (8), au moyen de laquelle du fluide à instiller peut être envoyé à partir du réservoir d'instillation (7) dans la cavité de plaie,
viii. un moyen pour envoyer un fluide à instiller du réservoir d'instillation (7) dans la cavité de plaie,
ix. un moyen de commande (5), dans lequel le moyen de commande (5) peut recevoir des valeurs de mesure de pression du moyen de capteur de pression (2), **caractérisé en ce que**
x. le moyen de commande peut émettre un signal pour arrêter la première unité de pompe (4) lorsqu'une valeur de seuil supérieure préréglée de la pression négative est atteinte dans la cavité de plaie, et
xi. le moyen de commande peut émettre un signal pour interrompre l'envoi du fluide à instiller lorsqu'une valeur de seuil inférieure préréglée de la pression négative est atteinte dans la cavité de plaie.

2. Dispositif pour la thérapie des plaies (20, 40, 50, 60) selon la revendication 1, comprenant en outre un moyen de détermination du volume de fluide à instiller, qui est introduit dans la cavité de plaie.

3. Dispositif pour la thérapie des plaies (20, 50, 60) selon la revendication 1 ou 2, dans lequel le moyen pour envoyer un fluide à instiller du réservoir d'instillation (7) dans la cavité de plaie est une seconde unité de pompe (9), qui est reliée en conduite de fluide au réservoir d'instillation (7).

4. Dispositif pour la thérapie des plaies (40) selon l'une quelconque des revendications précédentes, comprenant en outre une soupape (41), qui peut fermer la liaison de conduite de fluide entre le réservoir d'instillation (7) et la cavité de plaie, dans lequel le signal émis par le moyen de commande pour interrompre l'envoi du fluide à instiller provoque la fermeture de la soupape (41).

5. Dispositif pour la thérapie des plaies (20, 40, 50, 60) selon l'une quelconque des revendications précédentes, dans lequel le moyen de capteur de pression (2) est disposé dans la cavité de plaie.

6. Dispositif pour la thérapie des plaies (20, 40, 50, 60) selon l'une quelconque des revendications précédentes, dans lequel le moyen de capteur de pression (2) est en communication fluidique avec la conduite de drainage (3) et/ou le réservoir de drainage (1).

7. Dispositif pour la thérapie des plaies (20, 40, 50, 60) selon l'une quelconque des revendications précédentes, dans lequel le réservoir de drainage (1) est disposé dans le chemin de fluide entre la première unité de pompe (4) et la conduite de drainage (3).

8. Dispositif pour la thérapie des plaies (50) selon l'une quelconque des revendications précédentes, dans lequel le moyen de capteur de pression (2) est disposé dans le chemin de fluide entre la première unité de pompe (4) et le réservoir de drainage (1).

9. Dispositif pour la thérapie des plaies (60) selon l'une quelconque des revendications précédentes, dans lequel le dispositif pour la thérapie des plaies présente une conduite de mesure de pression séparée (61) et dans lequel la conduite de mesure de pression (61) est en communication fluidique avec le pansement à pression négative (15) et avec le moyen de capteur de pression (2) .

10. Dispositif pour la thérapie des plaies (20, 40, 50) selon l'une quelconque des revendications précédentes, dans lequel le dispositif pour la thérapie des plaies présente une conduite d'aération ou une conduite de rinçage (21) et dans lequel la conduite d'aération ou la conduite de rinçage (21) est en communication fluidique avec le moyen de capteur de pression (2).

11. Dispositif pour la thérapie des plaies (20, 40, 50, 60) selon l'une quelconque des revendications précédentes, dans lequel la valeur de seuil inférieure de la pression négative dans la cavité de plaie se situe entre 0 Pa (0 mm Hg) et 3999, 66 Pa (30 mm Hg), de préférence entre 666,61 Pa (5 mm Hg) et 3333, 05 Pa (25 mm Hg) et de préférence encore entre 1333,22 Pa (10 mm Hg) et 2666, 44 Pa (20 mm Hg).

12. Dispositif pour la thérapie des plaies (20, 40, 50, 60) selon l'une quelconque des revendications précédentes, dans lequel la valeur de seuil supérieure de la pression négative dans la cavité de plaie se situe entre 13332,2 Pa (100 mm Hg) et 17331,86 Pa (130 mm Hg), en particulier entre 15998,64 Pa (120 mm Hg) et 17331, 86 Pa (130 mm Hg).

13. Dispositif pour la thérapie des plaies (20, 40, 50, 60) selon l'une quelconque des revendications précédentes, dans lequel le dispositif pour la thérapie des plaies est sédentaire ou portable.

14. Dispositif pour la thérapie des plaies (20, 40, 50, 60) selon l'une quelconque des revendications précédentes, dans lequel le dispositif pour la thérapie des plaies présente un élément de régulation de la température (12) pour la régulation de la température du fluide à instiller.

15. Dispositif pour la thérapie des plaies (20, 40, 50, 60) selon la revendication 14, dans lequel l'élément de régulation de la température (12) comprend un élément chauffant à circulation.

16. Dispositif pour la thérapie des plaies (20, 40, 50, 60) selon l'une quelconque des revendications précédentes, dans lequel le fluide à instiller comprend un liquide.

17. Dispositif pour la thérapie des plaies (20, 40, 50, 60) selon la revendication 16, dans lequel le liquide est choisi parmi l'eau, une solution électrolytique ou une solution de Ringer.

18. Dispositif pour la thérapie des plaies (20, 40, 50, 60) selon l'une quelconque des revendications précédentes, dans lequel le fluide à instiller comprend un gaz.

19. Dispositif pour la thérapie des plaies (20, 40, 50, 60) selon la revendication 18, dans lequel le gaz est choisi parmi les gaz air, air enrichi en oxygène ou azote.
